# EUROPEAN PATENT SPECIFICATION

(11) **EP 2 369 944 B1**
(45) Date of publication and mention of the grant of the patent: **26.11.2014**
(21) Application number: 09796103.1
(22) Date of filing: 29.12.2009
(51) Int. Cl.: A23J 3/08, A23L 1/305, A61K 9/16, A61P 3/04

(54) **PROTEIN PARTICLES AND THEIR USE IN FOOD**
PROTEINPARTIKEL UND DEREN VERWENDUNG IN LEBENSMITTELN
PARTICULES DE PROTÉINE ET LEUR UTILISATION DANS LES ALIMENTS

(30) Priority: 29.12.2008 EP 08172995
(43) Date of publication of application: 05.10.2011
(73) Proprietor: Friesland Brands B.V., 3818 LE Amersfoort (NL)
(72) Inventor: VENEMA, Paul, NL-6701 EC Wageningen (NL); SAGIS, Leonard, Martin, Cornelia, NL-6708 BC Wageningen (NL); BOOIJ, Tieneke, Willemijn, NL-6709 RX Wageningen (NL); DE VRIES, Rindert, Jakob, NL-6866 EV Heelsum (NL); VAN DER LINDEN, Erik, NL-6717 XE Ede (NL)
(74) Representative: Nederlandsch Octrooibureau
(86) International application number: PCT/NL2009/050809
(87) International publication number: WO 2010/077137

(56) References cited:
- EP-A- 0 352 144
- EP-A- 0 412 590
- US-A- 4 734 287
- US-A- 5 897 896
- US-A1- 2002 039 617
- US-A1- 2008 220 071
- US-B2- 6 824 810

## Description

### Field of the invention

The invention relates to oil-comprising protein particles and a method for preparation thereof. The invention is particularly concerned with the use of such protein particles for increasing the protein content of a food product. Alternatively, the protein particles may be used for replacing fat in a food product, or for controlled release of protein in the gastrointestinal tract.

### Background

At present, food products with high protein content are highly in demand. Foods having high protein content can fulfil important functions with respect to human health. For instance, due to their relatively strong satiating effect in comparison to fats and carbohydrates, they can be used to help prevent or reduce over-eating and may play a key role in body-weight regulation and hence in treatment/prevention of obesity. Furthermore, they can help to prevent loss of muscular mass in elderly people (sarcopenia). For such purposes, protein levels in the food are required that are significantly higher than currently available. However, such high protein food systems are prone to very extensive protein-protein interactions resulting in very strong and tight aggregates/networks, exclusion of water from such structures and a tendency to further aggregate and phase separate. These phenomena will lead to textures that are experienced by consumers as being rubbery, tough and having a dry mouth feel. Moreover, the phenomena will lead to loss of storage stability.

US4734287 describes a product comprising a proteinaceous, water-dispersible, macrocolloid comprising substantially non-aggregated particles of dairy whey protein coagulate having mean diameter particle size distributions, when dried, ranging from about 0.1 microns to about 2.0 microns, with less than about 2 per cent of the total number of particles exceeding 3.0 microns in diameter, and wherein the majority of the said particles appear to be spheroidal when viewed at about 800 power magnification under a standard light microscope, whereby the colloid has a substantially smooth, emulsion-like organoleptic character when hydrated. There is also provided a process whereby the above described product may be produced.

US2002039617 describes insoluble, denatured, heat-stable protein particles used in food and beverage products. The particles are easily dispersible in aqueous solutions and take the form of substantially non-aggregated macro-colloids. In a hydrated state the preferred mean diameter particle size distribution of the insoluble, denatured, heat-stable particles ranges from about 0.1 microns to about 3.0 microns, with less than about 5 percent of the total number of particles exceeding about 3.0 microns in diameter. The majority of the particles are substantially spheroidal in shape and have a substantially smooth, emulsion-like organoleptic character similar to that of high-calorie fats and oils. Additionally, these particles have a degree of protein insolubility of at least about 80%, which provides processing advantages during heat treatment.

EP0352144 describes a method of preparing an aqueous composition of essentially non-aggregated colloidal particles of denatured heat-set storage protein, suitable for replacing at least part of the fat in food products, the process comprising heating an aqueous composition of insoluble undenatured heat-settable storage protein at a temperature sufficient to denature the protein and thereafter cooling below such temperature, while applying conditions of sufficient shear to prevent protein aggregation.

EP0412590 describes compositions, enriched in denatured alpha-lactalbumin, which are obtained by a process for denaturing these lactalbumins and separating these from the undenatured protein. These compositions can be used as fat replacer in food compositions.

US2004062846 describes powdered and liquid, dairy and non-dairy creamer compositions. These creamer compositions can be prepared in both concentrated and ready-to-use forms. The powdered creamer compositions are well suited for use in instant and/or dry food and beverage compositions that require the addition of water or other suitable fluids prior to use. The creamer compositions comprise a microparticulated ingredient component and a secondary ingredient component. The microparticulated ingredient component comprises a fat/oil component and a microparticulated protein component, and optionally a carbohydrate component. The secondary ingredient component of the creamer compositions of the present invention comprises an emulsifier and a bulking agent. The weight ratio of the microparticulated ingredient component to the secondary ingredient component is in the range of from about 1:99 to about 5:1, preferably in the range of from about 1:50 to about 5:1, more preferably in the range of from about 1:10 to about 5:1, even more preferably in the range of from about 1:5 to about 5:1.

US5897896 describes particles which provide a foodstuff binding agent which is dispersible in hot water and hot milk have a farinaceous or proteinaceous material core, an emulsifier coating layer about the core and a fat coating layer about the emulsifier layer, the emulsifier being, in particular, a phospholipid or a sucroglyceride and the fat being one which has a melting point of above 35 DEG C. The particles are prepared by dissolving an emulsifier in an apolar lipid composition, including a liquid oil, and spraying the lipid composition containing the emulsifier onto farinaceous and/or protein particles to coat the particles and thereafter, (a) melting an edible fat and spraying the melted fat onto the coated particles to coat the emulsifier-coated particles with the fat or and then, cooling the twice-coated particles, or (b) combining a ground particulate fat with the coated particles and coating the coated particles with the fat in the solid state. The particles may have a particle size in the range of 50-200 µm.

### Summary of the Invention

It is hypothesized that protein particles with high protein density can be used as building blocks and potentially also as steric blocks in high protein foods. Using such protein particles, palatable food products can be prepared with high protein content, and protein concentration can be decoupled from food structure or texture.

The present invention is concerned with the preparation of particles of high protein content that can be incorporated into a food product as to provide high protein content without negatively affecting the sensory properties of the food product.

Thus, in one aspect it is an object of the present invention to provide a W/O/W double emulsion method for preparing high-density protein particles that, upon addition to a food product, do not negatively affect the food product's sensory properties. The method comprises the steps of: a) providing an aqueous protein solution; b) providing an oil comprising a first emulsifier; c) providing a second emulsifier-comprising aqueous solution; d) preparing a primary emulsion by adding the protein solution to the oil comprising the first emulsifier and mixing the resultant to yield dispersed protein particles; e) preparing a secondary emulsion by homogenizing the primary emulsion of step d) in the second emulsifier-comprising aqueous solution; and f) separating oil-comprising protein particles obtained in step e) from the second emulsifier-comprising aqueous solution and optionally oil particles formed in the secondary emulsion.

In a further aspect, the invention is directed to oil-comprising protein particles obtainable by such method.

In another aspect, the invention is related to oil-comprising protein particles having a volume-to-surface mean particle diameter d₃₂ in the range of 0.5-12 µm, especially 1-10 µm and further comprising less than 2%, preferably less than 1%, (w/w) oil. In another aspect, the invention is related to oil-free protein particles having a volume-to-surface mean particle diameter d₃₂ in the range of 0.5-12 µm, especially 1-10 µm. Such oil-comprising protein particles may be obtainable by the method of the invention.

Also, the invention is concerned with the use of the protein particles according to the invention for increasing the protein content of a food product.

In yet a further aspect, the invention is concerned with the use of the protein particles according to the invention for (partially) replacing fat in a food product.

Moreover, the invention relates to the use of the protein particles according to the invention for controlled release of protein in the gastrointestinal tract of a subject.

### Detailed Description of Embodiments

For instance, in order to overcome sensory property problems in high protein food products, protein particles are provided that can be added to a food product to confer high protein level without negatively affecting the food product's sensory properties. The protein particles are also useful for replacing fat in a food product, and for controlled release of protein in the gastrointestinal tract.

In a first aspect of the present invention, a method is provided for preparing protein particles, said method comprising the steps of: a) providing an aqueous protein solution; b) providing an oil comprising a first emulsifier; c) providing a second emulsifier-comprising aqueous solution; d) preparing a primary emulsion by adding the protein solution to the oil comprising the first emulsifier and mixing the resultant to yield dispersed protein particles; e) preparing a secondary emulsion by mixing and homogenizing the primary emulsion of step d) in the second emulsifier-comprising aqueous solution; and f) separating oil-comprising protein particles obtained in step e) from the second emulsifier-comprising aqueous solution and optionally oil particles formed in the secondary emulsion.

In step a) an aqueous protein solution is provided. The term "protein" as used herein denotes a polypeptide having 10 or more amino acids, more preferably 20, 30, 40, 50, 70 or 100 or more amino acids, and can for instance be up to 500 or even 1000 amino acids, or even more. The protein solution may comprise one type of protein or several types of proteins. Moreover, the protein solution may comprise intact proteins or fractionated proteins, isolates, concentrates, or any combination thereof The protein incorporated in the particles may be any type of protein, but is preferably a food-grade protein. Examples of suitable food-grade proteins include, without limitation, dairy proteins, such as whey proteins (such as beta-lactoglobulins, alpha-lactalbumin, immunoglobulins), whey protein isolates, whey protein fractions, whey protein concentrates, casein, and milk proteins; vegetable proteins, such as soy proteins, soy protein isolates, soy protein fractions, soy protein concentrates; meat proteins; blood proteins; fish proteins; egg proteins; and the like. The person skilled in the art will be capable of identifying proteins that may be used for incorporation in food products. The protein concentration may be in the range of about 5 to about 60% (w/w), preferably about 10 to about 50% (w/w), such as about 20 to about 50% (w/w), about 25 to about 50% (w/w), about 30 to about 50% (w/w), or about 35 to about 50% (w/w). The pH of the aqueous protein solution is not critical, as long as the protein is soluble at the pH of the solution.

In step b) an oil is provided, which oil comprises a first emulsifier. The term "oil" as used herein includes fats and oils. The oil can be at least partially insoluble in an aqueous medium and/or is capable of forming an emulsion with an aqueous solution in the presence of a first emulsifier. The oil is preferably food-grade. Suitable oil includes, without limitation, naturally available oils or fats, purified fractions, and blends thereof. The oil may comprise one type of oil, or two or more types of oil, which altogether form a single oil phase. The oil may include any edible food-grade oil known to those skilled in the art. E.g., the oil may include an oil selected from the group consisting of vegetable oil such as corn oil, sunflower oil, soybean oil, canola oil, rapeseed oil, olive oil, nut oil, peanut oil, and algal oil; animal fat; and fish oil; or combinations (of two or more oils) thereof. Moreover, the oil may comprise any natural and/or synthetic lipid components, including, but not limited to, saturated and unsaturated fatty acids, glycerols, glycerides, phospholipids, glycolipids, phytosterol and/or sterol esters. Preferably, the oil is liquid at 20°C to 80°C, more preferably at 20°C to 50°C.

The oil may comprise a first emulsifier. The first emulsifier may comprise any emulsifier that is to some extent soluble in oil. The first emulsifier is preferably food-grade. Suitable first emulsifiers preferably have a low hydrophile-lipophile balance, HLB, number. This first emulsifier adsorbs to the surface of the oil droplets formed in the primary emulsion and forms a protective coating that prevents their subsequent aggregation. The skilled person is capable of selecting suitable first emulsifiers. It is to be noted that commercially available oil preparations may comprise emulsifiers as contaminants, and that in such cases no separate first emulsifier may be required. In a suitable embodiment, the emulsifier is a polyglycerol ester of polycondensed ricinoleic acid, hereinafter also referred to as "polyglycerol polyricinoleate", abbreviated hereinafter as "PGPR". The first emulsifier is present in a functionally effective amount. For PGPR, such functionally effective amount is in the range of about 1 to about 10% (w/w). The skilled person is capable of determining the functionally effective amount of an emulsifier.

In step c), a second emulsifier-comprising aqueous solution is provided. The composition of the aqueous solution is not critical, as long as it allows dispersion of oil droplets to obtain a secondary emulsion, as will be described below. The pH of the second emulsifier-comprising aqueous solution is not critical. The second emulsifier may be water-soluble, and may be food-grade. Non-limiting examples of such second emulsifier include caseinate salts, whey protein, lecithin, polysorbates (e.g. Tween 80), sorbitan esters, mono- and di-glycerides of fatty acids esters of monoglycerides of fatty acids and phosphated monoglycerides. The second emulsifier is present in a functionally effective amount. The skilled person is capable of determining the functionally effective amount of an emulsifier. E.g. in case of sodium caseinate, such functionally effective amount is in the range of about 0.5 to about 10% (w/w).

In step d), a primary emulsion (or W/O emulsion) is prepared from the oil and the aqueous protein solution. Thus, an emulsion is obtained with the aqueous protein solution being the internal (discontinuous) phase and the oil being the external (continuous) phase. The primary emulsion is prepared by mixing the oil and the aqueous protein solution. Preferably, mixing is carried out, e.g. using an Ultra-Turrax T25 with a speed below 20,000 RPM, preferably below 15,000 RPM, such as at 13,500 RPM or below, e.g. at 9,500 RPM or 6,500 RPM. The ratio of oil to aqueous protein solution in the primary emulsion may be in the range of about 95% : 5% (w/w) to about 55% : 45% (w/w). In some cases, about 15 to about 55% (w/w) aqueous protein solution is dispersed in the oil phase. Thus, dispersed protein particles are formed. By performing mixing, the dispersed protein particles may have a volume-to-surface mean particle diameter d₃₂, as measured by direct microscopy, CLSM, or static light scattering, in the range of about 0.5-12 µm, especially about 1 to 10 µm.

In step e), a secondary emulsion is prepared by homogenizing the primary emulsion of step d) in the second emulsifier-comprising aqueous solution. The secondary emulsion comprises oil droplets, which oil droplets in turn comprise dispersed protein particles. Thus, an inner aqueous phase, an outer aqueous phase, and an oil phase are comprised. In a preferred embodiment, an oil droplet comprises a single dispersed protein particle. It was found by the present inventors that the secondary emulsion may further comprise oil particles devoid of protein. One skilled in the art is well aware of suitable homogenization methods. These include, without limitation, a membrane homogenizer, or a high-pressure homogenizer.

In step f), separating oil-comprising protein particles obtained in step e) from the second emulsifier-comprising aqueous solution and optionally oil particles formed in the secondary emulsion. Said separation may be performed by any method known in the art, e.g. by centrifugation, or (micro)filtration. Subsequently, the oil-comprising protein particles may be dried. Drying may occur by any method known in the art, such as freeze-drying, vacuum-drying, spray-drying, and the like.

In an embodiment, the primary emulsion of step d) is treated to cause gelling of the proteins. Proteins generally can be cross-linked by means of heating, with sulphur bridges providing the cross-linking, such as for instance whey proteins like betalactoglobulin and alpha-lactalbumin. Moreover, proteins can be cross-linked by acidification or under the influence of an enzyme. Chemical cross-linking reactions, e.g. using a cross-linking agent, are also possible. In a very suitable embodiment, if globular proteins are present in the aqueous protein solutions, these are gelled by thermal gelation. Globular proteins, such as those from milk, egg, blood or soy, form gels when heated above their thermal denaturation temperature. For example, a primary emulsion comprising whey protein may be heated to about 80°C for about 20 minutes to ensure cross-linking of the protein, prior to preparation of the secondary emulsion.

Prior to preparation of the secondary emulsion, excess oil may be removed from the primary emulsion, e.g. by centrifuging the primary emulsion and removal of the supernatant, or by microfiltration. Any separation method known in the art may be used to remove excess oil.

The oil-comprising protein particles of step f) may be washed with an aqueous wash solution and may subsequently be separated from the aqueous wash solution. The composition of the wash solution is not critical as long as it is an aqueous solution.

In an embodiment the wash solution comprises or consists of second emulsifier-comprising aqueous solution. The wash step may be repeated. E.g., good results are obtained when the oil-comprising protein particles are washed at least twice using second emulsifier-comprising aqueous solution. The resultant is an oil-comprising protein particle, which may be dried using conventional techniques as described hereinabove. The amount of oil that is removed will depend on whether the wash solution comprises an emulsifier, which emulsifier, which concentration of emulsifier, and the number of wash steps applied.

Highly suitable double emulsions are obtained when the first emulsifier comprises polyglycerol polyricinoleate. Polyglycerol polyricinoleate is commercially available. Examples of such commercially available PGPR include, without limitation, Admul WOL (Quest, The Netherlands), and Triodan R90 (Grindsted, Danisco A/S, Denmark).

In a preferred embodiment, in step d) the protein solution and the oil are mixed at a speed of below 20,000 RPM, such as below 15,000 RPM. It is preferable to mix at such speeds in order to prevent the formation of protein particles having a volume-to-surface mean particle diameter d₃₂ smaller than 1 µm. It is highly preferable that no homogenization is carried out in the preparation of the primary emulsion in order to prevent the formation of protein particles having a volume-to-surface mean particle diameter d₃₂ smaller than approximately 1 µm. Thus, mixing according to the invention should be carried out in such a way that protein particles having a volume-to-surface mean particle diameter d₃₂ of between about 0.5-12 µm, especially about 1 and 10 µm are formed. Mixing may be carried out by any mixing device known in the art. In a preferred embodiment, mixing is carried out using an Ultra-Turrax, e.g. an Ultra-Turrax T25, at 6,500, 9,500 or 13,500 RPM.

In a further aspect, the present invention relates to a protein particle obtainable by a method as defined hereinabove. Such protein particle mainly has the characteristics described below. It is further expected that such protein particle may comprise a thin outer layer of oil, which may be visualised, e.g. by Scanning Electron Microscopy (SEM), or CARS-CLSM.

In another aspect, the invention is directed to an oil-comprising protein particle having a volume-to-surface mean particle diameter d₃₂ in the range of 0.5-12 µm, especially 1-10 µm, and further comprising less than 2%, e.g. about 0.01 to about 2% (w/w), preferably less than 1%, (w/w) oil. The oil content may be determined using any method known in the art. A non-limiting example of such method is the Soxlett-method. In an embodiment, said oil may be distributed homogeneously throughout the particle. The phrase "homogeneously distributed throughout the particle" refers to even distribution of oil over the protein particle as can be evidenced visually using Confocal Light Scanning Microscopy (CLSM) using a colorant, e.g., Nile Red, to colour the oil, or coherent anti-Stokes Raman Scattering (CARS)-CLSM.

The phrases "distributed throughout the particle" and "distributed throughout the particles" and similar phrases, are herein used to indicate that the oil is distributed throughout a particle. Alternatively, the phrase "distributed within the particle" may be applied.

The volume-to-surface mean particle diameter d₃₂ may be determined using for example laser diffraction. One skilled in the art is capable of determining the d₃₂.

As mentioned above, the d₃₂ value of the particles is preferably in the range of 0.5-12 µm, especially 1-10 µm. A desired particle size may also be defined as (oil-comprising or oil-free) protein particles wherein 80 % or more, preferably 90 % or more, of the particles have dimensions in the range of about 0.5-12 µm, especially about 1-10 µm. The dimensions (such as diameter in the case of substantially spherical particles) and the relative numbers of particles fulfilling the criteria may for instance be derived from microscopy, CLSM (or SEM) measurements (of the food product containing the particles of the invention).

The protein particle is substantially spherical. The protein in the particle may have gelled, e.g. due to thermal denaturation of the protein as described hereinabove with reference to the preparation method. In an alternative embodiment, the protein particle has a core-shell morphology. The core comprises protein and optionally a small amount of oil, whereas the shell is formed by an oil layer. The core-shell morphology may be observed in particular using CARS-CLSM. In case the core comprises some oil, the oil is not necessarily distributed homogeneously throughout the core. For example, in some cases it has been observed with CARS-CLSM that the core comprises locations not containing oil even though oil was present in the core.

In yet a further embodiment, there are provided oil-comprising protein particles, wherein the particles comprise a core comprising protein and patches of oil coated on the core, wherein at least 5% of the core is coated with the oil patches, such as in the range of 5-80% of the core. In a specific embodiment, more than 80% of the core of the particle is coated, such as up to 90%. Over 90%, the morphology can be considered of the core-shell type, since substantially the whole core is coated with oil. The presence of oil on the core does not exclude the concomitant presence of oil in the core.

Hence, in an embodiment, oil-comprising protein particles are provided, wherein said particles comprise a core comprising protein and patches of oil coated on the core, wherein in the range of 5-80% of the core is coated with the oil patches. In yet another embodiment, oil-comprising protein particles are provided, wherein said particles comprise an inhomogeneous distribution of oil throughout the particle (i.e. in an embodiment heterogeneously distributed in the "core"). Hence, in embodiments the oil may be distributed heterogeneously over the surface of the particle and/or throughout the particle.

With the method of the invention, it is also possible to provide oil-comprising protein particles with particles sizes (d₃₂) in more narrow ranges, such as for instance 0.5 - 2 µm or 1 - 2.5 µm, 2 -5 µm, 4 - 10 µm, etc. Likewise, at least 80%, preferably at least 90% of the particles may have a particle size in such range.

The particles according to the invention may also be dried, such as to remove at least part of the water. Drying may be performed with methods known in the art, such as freeze-drying or spray-drying or fluid-bed drying, etc. Therefore, the invention also involves a method further comprising drying the (oil-comprising or oil-free) protein particles. Drying may be performed after separating oil-comprising protein particles or after an oil-extraction process to provided oil-free protein particles, as described herein.

Hence, in another aspect, the invention is related to dried oil-comprising protein particles (preferably having a volume-to-surface mean particle diameter d₃₂ in the range of 0.5-12 µm, especially 1-10 µm). Such oil-comprising or oil-free protein particles may be obtainable by the method of the invention, followed by a drying process. In a further specific embodiment, the invention provides lyophilized oil-free or alternatively oil-containing particles.

The invention also provides dried oil-comprising protein particles (preferably having a volume-to-surface mean particle diameter d₃₂ in the range of 0.5-12 µm, especially 1-10 µm) comprising less than 12%, preferably less than 8%, (w/w) oil, even more preferably less than 6%, (w/w) oil.

In a specific embodiment, the invention provides oil-comprising protein particles (especially obtainable by a method including drying), comprising about 0.01 to about 12% (w/w) oil, preferably about 0.01 to about 8%, (w/w) oil, such as 0.01 to about 6%, (w/w) oil. In a specific embodiment, the oil content is at least 0.02 %, such as 0.05 % (w/w) oil (for those dried particles).

In general, in food products, the particles will be hydrated again. (Re)hydrated particles have again, when comprising oil, about 0.01 to about 2 (w/w) oil.

Also, the invention encompasses a food product comprising a protein particle as defined hereinabove. The protein particle may comprise some oil or may be oil-free. It is envisioned that the addition of such protein particles does not negatively affect the sensory properties of said food product. The food product may be any food product such as a dairy product, a meat product, meat replacers, processed meat products, sausages, nutritional bars, mashed potato products, snacks, processed cheese, cheese, baked, fried or cooked dough products, or any other food product. The protein particles may also be used to replace fat, e.g. in hot and cold sauces.

Envisioned is the use of a protein particle as herein described for increasing the protein content of a food product. The association of the protein particle with the oil particularly allows incorporation thereof in a food product without negatively affecting its sensory properties such as texture.

In another aspect, the protein particles as herein described are used for replacing fat in a food product. In particular the oil-comprising protein particles have an external structure resembling fat globules and will as such confer the mouth feel of fat globules whilst having a protein internal phase rather than a fat internal phase. Such food products are particularly useful for reducing the calorie intake of a subject. Suitable food products include hot and cold sauces, and dairy products.

Alternatively, the protein particles as herein described may be used for controlled release of protein in the gastrointestinal tract, in particular in the gastrointestinal tract of a subject.

It will also be clear that the description and examples are included merely to illustrate some embodiments of the invention, and not to limit the scope of protection. Starting from this disclosure, many more embodiments will be evident to a skilled person, which are within the scope of protection and the essence of this invention and which are obvious combinations of prior art techniques and the disclosure of this patent.

### Examples

### Example 1. Preparation of whey protein containing particles in sunflower oil

A 25 % (w/w) whey protein solution (whey protein, WPI, Bipro, in Millipore water), pH 6.50, was prepared. 2.5 % (w/w) polyglycerol polyricinoleate (PGPR, Danisco) was dissolved in sunflower oil (Reddy). Also, a 4 % (w/w) sodium caseinate (hereinafter also referred to as "NaCas") solution (sodium caseinate powder, DMV International, in Millipore water), pH 6.88, was prepared.

A primary water-in-oil (w/o) emulsion (30% water-in-oil) was prepared by slowly adding 60 grams of 25 % (w/w) whey protein solution into 140 grams oil containing 2.5 % PGPR while mixing with an Ultra-Turrax T25 (6500 RPM, 5 minutes). Then, the emulsion was heated at 80 °C for 20 minutes whilst stirring. The resulting emulsion was centrifuged at 30,000g for 30 min, and the excess of oil (present as a supernatant) was decanted.

A secondary water-in-oil-in-water (w/o/w) emulsion was prepared as follows: The pellet (70 grams) was re-dispersed in the 4% (w/w) NaCas solution (140 grams), with the weight ratio of pellet to NaCas solution being 1:2. The dispersion was stirred gently and subsequently mixed with an Ultra-Turrax T25 at 6,500 RPM for 5 minutes. Next, the sample was homogenized in a lab scale homogenizer van Delta Instruments, Drachten, The Netherlands, at 150 bar, 6 passes. The homogenate was centrifuged for 30 minutes at 30,000g to remove oil droplets not comprising protein. After centrifugation, 3 layers were visible: a top/cream layer, a middle layer, and a pellet. The top/cream layer was removed and the pellet was re-dispersed with the middle liquid layer in the centrifugation tube. Next, the sample was mixed for 5 minutes at 6,500 RPM using an Ultra-Turrax T25. The centrifugation and re-dispersing step was repeated at the same conditions, and finally the sample was homogenized in a lab scale homogenizer van Delta Instruments, Drachten, The Netherlands, at 150 bar, 2 passes.

To characterize the whey protein containing particles, direct field microscopy, confocal light scanning microscopy (CLSM) and static light scattering (Master sizer, Malvern), were used. From these measurements it was concluded that the particles obtained are spherical, non-aggregated and have a typical diameter ranging between 700 nm and 10 microns. CLSM pictures demonstrated that the protein content was distributed homogeneously throughout the particles. CLSM also showed that oil was distributed homogenously throughout the particle. Although not visible by the CLSM method used, it is expected that a thin oil layer surrounded the protein core, imparting a core-shell morphology to the particle, with the core being made up mainly of the protein (albeit some oil may be present as well), and the shell being a thin oil layer.

The volume fraction of the protein particles in emulsion was determined by viscometry measurements. The volume fraction in combination with density measurements was used to determine the internal effective density of the protein particles. Moreover, the protein content (as determined by DUMAS) and fat content (as determined by Soxlett) of the particles was determined. From these measurements it was found that the internal protein fraction inside the protein particles was 17.82% (w/w), while the oil fraction in the protein particles was 1.3 % (w/w). The effective density of the protein particle was found to be 1.04377 kg/m³.

It was shown that the use of either whey protein isolate or whey protein concentrate (WPC 80) results in similar particles.

### Example 2. Protein particles in model food product

Particles were prepares as described in example 1. The pellet was redispersed at a concentration of 5% w/w, pH 5.7 and mixed with a 15%w/w WPI solution (see example 1), pH 5.7. The mixture was treated with an ultraturrax for 5 min at 6500 min-1 and subsequently homogenized at 150 bar (see example 1). Then a heat set gel was formed by heating for 10 min at 85°C in a water bath.

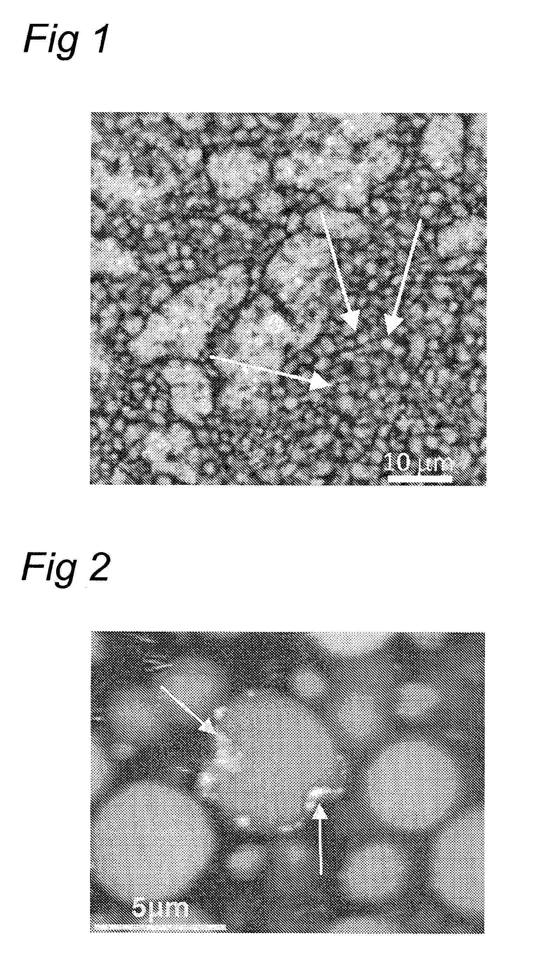

The gel was characterized with confocal light scanning microscopy (CLSM; figure 1). The (small) particles are clearly visible in the gel (and are indicated with arrows). In a reference gel without protein particles only the large gel fragments are visible (not shown).

### Example 3. Protein particles in food product

A luncheon meat product was made, wherein protein particles according to the invention were added. This product contained 27 wt.% fat and 3 wt% particles. The reference product contained 30 wt.% fat. Previous work showed that 27% fat plus 3% free protein (not in the form of the particles of the invention) leads to an unacceptable hard product.

CLSM characterization did not reveal differences in the microstructure of the sausages.

### Example 4. Protein particles synthesis route

An adapted synthesis route was developed, wherein the particles can be made on a kg scale. 30wt% of a WPI-protein-solution (25wt% WPI) was mixed with 70 wt% sunflower oil containing 2.5 % (w/w) polyglycerol polyricinoleate (PGPR, Danisco) in a batch size 80L. Pre-emulsions were in four 20L batches made by mild turrax treatment (Ultra-turrax T50 basic, Ika Werke, Germany); 7600 rpm, 5 min and subsequently heated in a heating-jacked funnel for 20 min 80°C (80L). Particles were harvested by centrifugation (6L; Centrifuge RC3C, Sorvall Instruments, US), washed (see example 1; last time pellet redispersed in water) and homogenized in 3 cycles (1 x 0 bar, 2 x 150 bar (positive pump; Homogenizer Lab100 115 300 X, Manton Gaulin). The obtained emulsions were pasteurized (20 sec 73-75°C, Combitherm, indirect continuous heating) and freeze-dried.

After redispersing the particles in water, the microstructure of the particles was characterized with CLSM. The same microstructures were obtained compared to the particle made according to the method described in example 1.

### Example 5. Protein particles measurements

Protein particles were characterized with CLSM and NMR.

For CLSM the oil phase was stained with Nile red or Nile blue, and the protein was covalently stained with FITC. CLSM images show the oil on the surface of the particles, possibly also throughout the particle (Fig YYY).

1H/13C-NMR spectra were recorded on a Bruker 500 MHz Ultrashield Avance III system equipped with ATM/TCI probe. The hydrated particles contained 20% protein and 1.1 % lipid.

Both CLSM and NMR data show the presence of oil associated with the particles. Figure 2 shows a CLSM figure, wherein the arrows indicate oil patches on the surface of the protein cores.

Use of the verb "to comprise" and its conjugations does not exclude the presence of elements or steps other than those stated in a claim. The article "a" or "an" preceding an element does not exclude the presence of a plurality of such elements. In the device claim enumerating several means, several of these means may be embodied by one and the same item of hardware.

## Claims

1. A method for preparing protein particles, said method comprising the steps of:
a) Providing an aqueous protein solution;
b) Providing an oil comprising a first emulsifier;
c) Providing a second emulsifier-comprising aqueous solution;
d) Preparing a primary emulsion by adding the protein solution to the oil comprising the first emulsifier and mixing the resultant to yield dispersed protein particles;
e) Preparing a secondary emulsion by homogenizing the primary emulsion of step d) in the second emulsifier-comprising aqueous solution; and
f) Separating oil-comprising protein particles obtained in step e) from the second emulsifier-comprising aqueous solution and optionally oil particles formed in the secondary emulsion.

2. A method according to claim 1, further comprising the step of treating the primary emulsion to cause gelling of the protein.

3. A method according to any of claims 1 or 2, further comprising the step of removing excess oil from the primary emulsion prior to preparation of the secondary emulsion.

4. A method according to any of the preceding claims, further comprising the step of washing the oil-comprising protein particles with an organic solvent to obtain oil-free protein particles.

5. A method according to any of the preceding claims, further comprising the step of drying the oil-comprising protein particles or oil-free protein particles.

6. A method according to any of the preceding claims, wherein the first emulsifier comprises polyglycerol polyricinoleate.

7. Oil-comprising protein particles obtainable by a method according to any of claims 1 - 6, wherein said oil is distributed homogeneously throughout the particles.

8. Oil-comprising protein particles obtainable by a method according to any of claims 1 - 6, said particles comprising a core comprising protein and patches of oil coated on the core, wherein in the range of 5-80% of the core is coated with the oil patches.

9. Oil-comprising protein particles according to claim 8, said particles comprising an inhomogeneous distribution of oil throughout the particle.

10. Oil-comprising protein particles according to any one of claims 7 - 9, having a volume-to-surface mean particle diameter d₃₂ in the range of 0.5-12 µm.

11. Oil-comprising protein particles according to any one of claims 7 - 10, wherein 80 % or more, preferably 90 % or more, of the particles have dimensions in the range of about 0.5-12 µm.

12. Oil-comprising protein particles having a volume-to-surface mean particle diameter d₃₂ in the range of 0.5-12 µm and further comprising about 0.01 to about 2% (w/w) oil, preferably about 0.01 to about 1% (w/w) oil.

13. Oil-comprising protein particles according to claim 12, wherein said oil is distributed homogeneously throughout the particles.

14. Oil-comprising protein particles according to any one of claims 12 - 13, said particles having a core-shell morphology with the core comprising protein and the shell substantially consisting of oil.

15. Oil-comprising protein particles according to any one of claims 12 - 14, said particles comprising a core comprising protein and patches of oil coated on the core, wherein in the range of 5-80% of the core is coated with the oil patches.

16. Oil-comprising protein particles according to any one of claims 12 and 14 - 15, said particles comprising an inhomogeneous distribution of oil throughout the particle.

17. Food product comprising protein particles according to any of claims 7 - 16.

18. Use of protein particles according to any of claims 7 - 16 for increasing the protein content of a food product.

19. Use of protein particles according to any of claims 7 - 16 for replacing fat in a food product.

## Patentansprüche

1. Verfahren zur Herstellung von Proteinpartikeln, bei dem
a) eine wässrige Proteinlösung zur Verfügung gestellt wird,
b) ein Öl, das einen ersten Emulgator umfasst, zur Verfügung gestellt wird,
c) eine wässrige Lösung, die einen zweiten Emulgator umfasst, zur Verfügung gestellt wird,
d) eine primäre Emulsion hergestellt wird, indem die Proteinlösung zu dem Öl, das den ersten Emulgator umfasst, gegeben wird und die sich daraus ergebende Mischung gemischt wird, um dispergierte Proteinpartikel zu erhalten,
e) eine sekundäre Emulsion hergestellt wird, indem die primäre Emulsion aus Schritt d) in der wässrigen Lösung, die den zweiten Emulgator umfasst, homogenisiert wird und
f) Öl-enthaltende Proteinpartikel aus Schritt e) von der wässrigen Lösung, die den zweiten Emulgator umfasst, und gegebenenfalls von in der sekundären Emulsion gebildeten Ölpartikeln getrennt werden.

2. Verfahren nach Anspruch 1, bei dem ferner die primäre Emulsion behandelt wird, um Gelieren des Proteins zu bewirken.

3. Verfahren nach einem der Ansprüche 1 oder 2, bei dem ferner vor dem Herstellen der sekundären Emulsion das überschüssige Öl von der primären Emulsion entfernt wird.

4. Verfahren nach einem der vorhergehenden Ansprüche, bei dem ferner die Öl-enthaltenden Proteinpartikel mit einem organischen Lösemittel gewaschen werden, um Öl-freie Proteinpartikel zu erhalten.

5. Verfahren nach einem der vorhergehenden Ansprüche, bei dem ferner die Öl-enthaltenden oder Öl-freien Proteinpartikel getrocknet werden.

6. Verfahren nach einem der vorhergehenden Ansprüche, bei dem der erste Emulgator Polyglycerin-Polyricinoleat umfasst.

7. Öl-enthaltende Proteinpartikel, erhältlich durch ein Verfahren nach einem der Ansprüche 1-6, wobei das Öl homogen über die Partikel verteilt ist.

8. Öl-enthaltende Proteinpartikel, erhältlich durch ein Verfahren nach einem der Ansprüche 1-6, die einen Kern, der Protein umfasst, und den Kern bedeckende Ölflecke umfassen, wobei 5-80% des Kerns mit den Ölflecken bedeckt sind.

9. Öl-enthaltende Proteinpartikel nach Anspruch 8, wobei die Partikel eine inhomogene Verteilung des Öls innerhalb eines Partikels aufweisen.

10. Öl-enthaltende Proteinpartikel nach einem der Ansprüche 7-9, die einen mittleren Volumen-Oberflächendurchmesser der Partikel d₃₂ im Bereich von 0,5-12 µm aufweisen.

11. Öl-enthaltende Proteinpartikel nach einem der Ansprüche 7-10, wobei 80% oder mehr, bevorzugt 90% oder mehr, der Partikel eine Größe im Bereich von ungefähr 0,5-12µm aufweisen.

12. Öl-enthaltende Proteinpartikel, die einen mittleren Volumen-Oberflächendurchmesser der Partikel d₃₂ im Bereich von 0,5-12 µm aufweisen und ferner ungefähr 0,01 bis ungefähr 2% (Gew./Gew.) Öl, bevorzugt ungefähr 0,01 bis ungefähr 1% (Gew./Gew.) Öl, umfassen.

13. Öl-enthaltende Proteinpartikel nach Anspruch 12, wobei das Öl homogen über die Partikeln verteilt ist.

14. Öl-enthaltende Proteinpartikel nach einem der Ansprüche 12-13, die eine Kern/Schalen-Morphologie aufweisen, wobei der Kern Protein umfasst und die Schale im Wesentlichen aus Öl besteht.

15. Öl-enthaltende Proteinpartikel nach einem der Ansprüche 12-14, die einen Kern, der Protein umfasst, und den Kern bedeckende Ölflecke umfassen, wobei 5-80% des Kerns mit den Ölflecken bedeckt sind.

16. Öl-enthaltende Proteinpartikel nach einem der Ansprüche 12 und 14-15, wobei die Partikel eine inhomogene Verteilung des Öls innerhalb eines Partikels aufweisen.

17. Nahrungsmittelprodukt, das Proteinpartikel gemäß einem der Ansprüche 7-16 umfasst.

18. Verwendung von Proteinpartikeln gemäß einem der Ansprüche 7-16 zur Erhöhung des Proteinanteils eines Nahrungsmittelprodukts.

19. Verwendung von Proteinpartikeln gemäß einem der Ansprüche 7-16 zum Ersetzen von Fett in einem Nahrungsmittelprodukt.

## Revendications

1. Procédé de préparation de particules de protéine, ledit procédé comprenant les étapes suivantes :
a) la fourniture d'une solution aqueuse de protéine ;
b) la fourniture d'une huile comprenant un premier émulsifiant ;
c) la fourniture d'une deuxième solution aqueuse comprenant un émulsifiant ;
d) la préparation d'une émulsion primaire en ajoutant la solution de protéine à l'huile comprenant le premier émulsifiant et le mélange du produit obtenu pour donner des particules de protéine dispersées ;
e) la préparation d'une émulsion secondaire par homogénéisation de l'émulsion primaire de l'étape d) dans la deuxième solution aqueuse comprenant un émulsifiant ; et
f) la séparation de particules de protéine comprenant de l'huile obtenues à l'étape e) à partir de la deuxième solution aqueuse comprenant un émulsifiant et, éventuellement, des particules d'huile formées dans l'émulsion secondaire.

2. Procédé selon la revendication 1, comprenant en outre l'étape de traitement de l'émulsion primaire pour entraîner la gélification de la protéine.

3. Procédé selon l'une quelconque des revendications 1 ou 2, comprenant en outre l'étape de retrait de l'excédent d'huile de l'émulsion primaire avant la préparation de l'émulsion secondaire.

4. Procédé selon l'une quelconque des revendications précédentes, comprenant en outre l'étape de lavage des particules de protéine comprenant de l'huile, avec un solvant organique, pour obtenir des particules de protéine sans huile.

5. Procédé selon l'une quelconque des revendications précédentes, comprenant en outre l'étape de séchage des particules de protéine comprenant de l'huile ou des particules de protéine sans huile.

6. Procédé selon l'une quelconque des revendications précédentes, dans lequel le premier émulsifiant comprend du polyricinoléate de polyglycérol.

7. Particules de protéine comprenant de l'huile, pouvant être obtenues par un procédé selon l'une quelconque des revendications 1 à 6, dans lesquelles ladite huile est distribuée de façon homogène dans toutes les particules.

8. Particules de protéine comprenant de l'huile, pouvant être obtenues par un procédé selon l'une quelconque des revendications 1 à 6, lesdites particules comprenant une protéine comprenant un noyau et des patchs d'huile recouvrant le noyau, dans lesquelles un plage de 5 à 80 % du noyau est recouverte des patchs d'huile.

9. Particules de protéine comprenant de l'huile selon la revendication 8, lesdites particules comprenant une distribution non homogène de l'huile dans toutes les particules.

10. Particules de protéine comprenant de l'huile selon l'une quelconque des revendications 7 à 9, ayant un diamètre moyen de particule de volume à surface d₃₂ dans une plage de 0,5 à 12 µm.

11. Particules de protéine comprenant de l'huile, selon l'une quelconque des revendications 7 à 10, dans lesquelles 80 % ou plus, de préférence 90 % ou plus, des particules ont des dimensions dans une plage d'environ 0,5 à 12 µm.

12. Particules de protéine comprenant de l'huile, ayant un diamètre moyen de particule de volume à surface d₃₂ dans une plage de 0,5 à 12 µm et comprenant en outre environ 0,01 à environ 2 % (p/p) d'huile, de préférence environ 0,01 à environ 1 % (p/p) d'huile.

13. Particules de protéine comprenant de l'huile, selon la revendication 12, dans lesquelles ladite huile est distribuée de façon homogène dans toutes les particules.

14. Particules de protéine comprenant de l'huile selon l'une quelconque des revendications 12 à 13, lesdites particules ayant une morphologie noyau-coque, le noyau comprenant la protéine et la coque consistant sensiblement en huile.

15. Particules de protéine comprenant de l'huile selon l'une quelconque des revendications 12 à 14, lesdites particules comprenant un noyau comprenant une protéine et des patchs d'huile recouvrant le noyau, dans lesquelles une plage de 5 à 80 % du noyau est recouverte des patchs d'huile.

16. Particules de protéine comprenant de l'huile selon l'une quelconque des revendications 12 et 14 à 15, lesdites particules comprenant une distribution non homogène d'huile dans toutes les particules.

17. Produit alimentaire comprenant des particules de protéine selon l'une quelconque des revendications 7 à 16.

18. Utilisation de particules de protéine selon l'une quelconque des revendications 7 à 16, pour augmenter la teneur en protéine d'un produit alimentaire.

19. Utilisation de particules de protéine selon l'une quelconque des revendications 7 à 16, pour remplacer des matières grasses dans un produit alimentaire.
